# EUROPEAN PATENT APPLICATION

(11) **EP 4 209 262 A1**
(43) Date of publication of application: **12.07.2023**
(21) Application number: 22862404.5
(22) Date of filing: 05.07.2022
(51) Int. Cl.: B01F 23/231

(54) **BUBBLE COLUMN REACTOR**

(30) Priority: 23.11.2021 KR 20210162827
(71) Applicant: LG CHEM, LTD., Yeongdeungpo-gu, Seoul 07336 (KR)
(72) Inventor: HWANG, Moon Sub, Daejeon 34122 (KR); LEE, Jeong Seok, Daejeon 34122 (KR); LEE, Hong Min, Daejeon 34122 (KR)
(74) Representative: Hoffmann Eitle
(86) International application number: PCT/KR2022/009669
(87) International publication number: WO 2023/096049

(57) **Abstract**

Provided is a bubble column reactor including a down chamber formed at a lower portion, a liquid reaction zone formed at an upper portion of the down chamber, a dispersion plate formed between the down chamber and the reaction zone, and a gas supply pipe connected to the down chamber to supply a gaseous reactant, wherein the gas supply pipe includes a spray part extending into the down chamber, wherein the spray part includes a plurality of spray nozzles spraying the gaseous reactant to a lower side of the down chamber.

## Description

### [Technical Field]

### Cross-reference to related application

The present application claims priority to Korean Patent Application No. 10-2021-0162827, filed on November 23, 2021, the entire contents of which are incorporated herein for all purposes by this reference.

### Technical field

The present invention relates to a bubble column reactor, and more particularly, to a bubble column reactor for improving mixing efficiency of a reaction medium by uniformly supplying a gaseous reactant to a reaction zone when preparing an oligomer in the bubble column reactor.

### [Background Art]

Alpha-olefins are widely used commercially as important materials used in comonomers, detergents, lubricants, and plasticizers. In particular, 1-hexene and 1-octene have been widely used as comonomers for controlling the density of polyethylene in the production of linear low-density polyethylene (LLDPE).

Alpha olefins are typically prepared through oligomerization of ethylene. As a reactor type in which the ethylene oligomerization reaction is carried out, a bubble column reactor performing an oligomerization reaction of ethylene (trimerization reaction or tetramerization reaction) through contact with a reaction zone including a liquid reaction medium including a catalyst using gaseous ethylene as a reactant has been used.

In the bubble column reactor, a gaseous reactant is supplied to a down chamber provided at a lower portion of the reactor, introduced into a reaction zone including a liquid reaction medium, and simultaneously dispersed and mixed through a dispersion plate.

In the related art, in supplying the gaseous reactant to the down chamber through a gas supply pipe, a method of spraying upwardly toward the dispersion plate in the down chamber was adopted, but in this case, it was difficult for the gaseous reactant to pass through the dispersion plate in a sufficiently mixed state in the down chamber by a spray pressure. In addition, due to problems such as clogging of holes of the dispersion plate by various byproducts produced by an oligomerization reaction performed in the reaction zone, there was a limitation in uniformly supplying the gaseous reactant only by the dispersion plate. This resulted in a decrease in mixing efficiency between the reaction medium and the gaseous reactant in the reaction zone.

Therefore, in order to solve the above problems, there is a need for research into uniform dispersion and supply of a gaseous reactant supplied to the reaction zone.

### [Disclosure]

### [Technical Problem]

In order to solve the problems mentioned in the background of the present invention, an object of the present invention is to provide a bubble column reactor in which, before being supplied to a reaction zone, a gaseous reactant in a down chamber is sufficiently uniformly dispersed and then supplied to the reaction zone.

### [Technical Solution]

In one general aspect, a bubble column reactor includes: a down chamber formed at a lower portion, a liquid reaction zone formed at an upper portion of the down chamber, a dispersion plate formed between the down chamber and the reaction zone, and a gas supply pipe connected to the down chamber to supply a gaseous reactant, wherein the gas supply pipe includes a spray part extending into the down chamber, and the spray part includes a plurality of spray nozzles spraying the gaseous reactant to a lower side of the down chamber.

### [Advantageous Effects]

According to the bubble column reactor of the present invention, by spraying a gaseous reactant to the lower side of the down chamber through the spray part extending into the down chamber from the gas supply pipe, a path and time for the gaseous reactant to be sufficiently mixed in the down chamber may be provided and the gaseous reactant may be uniformly supplied into the reaction zone and mixed with a reaction medium.

In addition, by setting a spray direction and a spray angle to be different according to a position of the spray nozzle of the spray part, uniform mixing of the sprayed gaseous reactant may be achieved, thereby uniformly supplying the gaseous reactant in the reaction zone and maximizing the mixing efficiency of the reaction zone.

### [Description of Drawings]

FIG. 1 is a flowchart of a bubble column reactor and a related process according to an embodiment of the present invention.
FIG. 2 is an enlarged view of a portion of a bubble column reactor according to an embodiment of the present invention.
FIG. 3 is an enlarged view of a portion of a bubble column reactor according to another embodiment of the present invention.
FIG. 4 is a flowchart of a bubble column reactor and a related process according to the related art.

### [Best Mode]

The terms and words used in the description and claims of the present invention are not to be construed as general or dictionary meanings but are to be construed as meanings and concepts meeting the technical ideas of the present invention based on a principle that the inventors may appropriately define the concepts of terms in order to describe their own inventions in the best mode.

The term "stream" in the present invention may refer to a flow of a fluid in a process, and may also refer to a fluid flowing through a transfer line (pipe) itself. Specifically, the "stream" may refer to both the fluid flowing through the pipe connecting respective devices to each other itself and the flow of the fluid at the same time. In addition, the fluid may refer to one or more of a gas, a liquid, and a solid.

The term "C#" in which "#" is a positive integer in the present invention refers to all hydrocarbons having # carbon atoms. Accordingly, the term "C10" refers to a hydrocarbon compound having 10 carbon atoms. In addition, the term "C#+" refers to all hydrocarbon molecules having # or more carbon atoms. Accordingly, the term "C10+" refers to a mixture of hydrocarbons having 10 or more carbon atoms.

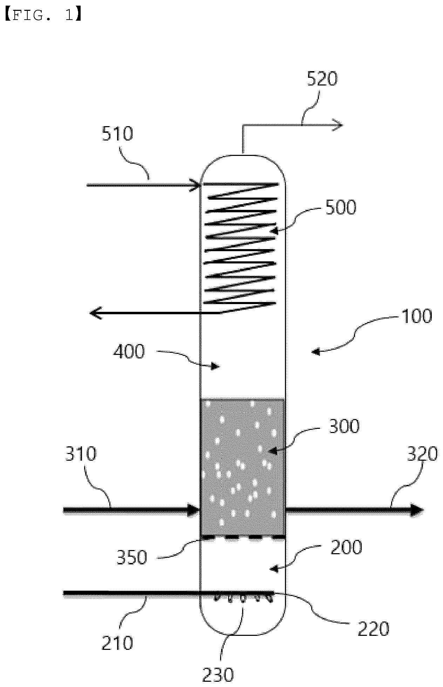

Hereinafter, the present invention will be described in more detail with reference to FIG. 1 in order to help the understanding of the present invention.

According to the present invention, a bubble column reactor 100 is provided. The bubble column reactor 100 includes a down chamber 200 formed at a lower portion, a liquid reaction zone 300 formed at an upper portion of the down chamber 200, a dispersion plate 350 formed between the down chamber and the reaction zone, and a gas supply pipe 210 connected to the down chamber to supply a gaseous reactant, wherein the gas supply pipe 210 includes a spray part 220 extending into the down chamber 200, and the spray part 220 includes a plurality of spray nozzles 230 spraying the gaseous reactant to a lower side of the down chamber 200.

According to an embodiment of the present invention, the bubble column reactor 100 may be for preparing an oligomer product by performing an oligomerization reaction on a monomer in the presence of a catalyst and a solvent.

More specifically, the bubble column reactor 100 may include the reaction zone 300, and through one or more reaction medium supply lines 310 connected to one side of the reaction zone 300, the reaction medium may be supplied to the reaction zone 300.

Meanwhile, the reaction medium may include a catalyst, a co-catalyst, and a solvent. The catalyst, the co-catalyst, and the solvent may be supplied through separate reaction medium supply lines 310, respectively, and two or more reaction medium components may be mixed and supplied to the reaction zone 300 through the reaction medium supply line 310.

According to an embodiment of the present invention, the monomer may include an ethylene monomer. As a specific example, the gaseous reactant including the ethylene monomer may be supplied into a down chamber 200 of the bubble column reactor 100 to be described later to produce a desired alpha olefin product through an oligomerization reaction.

The solvent may include one or more selected from the group consisting of n-pentane, n-hexane, n-heptane, cyclohexane, methylcyclohexane, octane, cyclooctane, decane, dodecane, benzene, xylene, 1,3,5-trimethylbenzene, toluene, ethylbenzene, chlorobenzene, dichlorobenzene, and trichlorobenzene.

The catalyst may include a transition metal source. The transition metal source may be a compound including one or more selected from the group consisting of, for example, chromium(III) acetylacetonate, chromium(III) chloride tetrahydrofuran, chromium(III) 2-ethylhexanoate, chromium(III) tris(2,2,6,6-tetramethyl-3,5-heptanedionate), chromium (III) benzoylacetonate, chromium (III) hexafluoro-2,4-pentaindionate, chromium (III) acetate hydroxide, chromium (III) acetate, chromium(III) butyrate, chromium(III) pentanoate, chromium(III) laurate, and chromium(III) stearate.

The co-catalyst may include one or more selected from the group consisting of, for example, trimethyl aluminum, triethyl aluminum, triisopropyl aluminum, triisobutyl aluminum, ethyl aluminum sesquichloride, diethylaluminum chloride, ethyl aluminum dichloride, methylaluminoxane, modified methylaluminoxane, and borate.

Meanwhile, in the reaction zone 300 of the bubble column reactor 100, the oligomerization reaction with the monomer may be performed in a liquid reaction medium including a catalyst, a co-catalyst, and a solvent. As described above, a zone composed of a reaction medium in which an oligomerization reaction of a monomer is performed may be defined as the reaction zone 300. The oligomerization reaction may refer to a reaction in which a monomer is oligomerized. Depending on the number of monomers to be polymerized, the oligomerization reaction may be called trimerization and tetramerization, which are collectively called multimerization.

The alpha olefin is widely used commercially as an important material used in a comonomer, detergent, lubricant, plasticizer, etc. In particular, 1-hexene and 1-octene are commonly used as a comonomer for controlling the density of polyethylene in the production of linear low-density polyethylene (LLDPE). The alpha olefins such as 1-hexene and 1-octene may be prepared through, for example, trimerization or tetramerization of ethylene.

The bubble column reactor 100 may include a product discharge line 320 connected to the reaction zone 300 and provided on the other side of the reaction medium supply line, and a product including alpha olefin, which is a product of the oligomerization reaction, may be discharged through the product discharge line. The supply of the reaction medium to the reaction zone 300 and the discharge of the product from the reaction zone 300 may be performed continuously.

Meanwhile, the gaseous reactant including the monomer for the oligomerization reaction may be supplied into the down chamber 200 located at the lower portion of the bubble column reactor 100, and then passed through a dispersion plate 350 to be supplied to the reaction zone 300 including the liquid reaction medium.

That is, the dispersion plate 350 may be provided between the down chamber 200 and the reaction zone 300, and the gaseous reactant, e.g., the monomer, may be uniformly distributed and supplied to the reaction zone 300 including the reaction medium from the down chamber 200 through holes formed at equal intervals along the center and circumference of the dispersion plate 350.

The gaseous reactant flows into the reaction zone 300 including the liquid reaction medium through the dispersion plate 350 and is dispersed at the same time, and turbulence is generated by a force of the dispersed gas, so that the liquid reaction medium and the gaseous reactant are naturally mixed. At this time, the dispersion force of the gaseous reactant flowing into the reaction zone 300 through the dispersion plate 350 may be maintained to be greater than a head pressure acting downwardly from the liquid reaction medium, so that the liquid reaction medium may remain in the reaction zone 300.

According to an embodiment of the present invention, the gaseous reactant may be supplied into the down chamber 200 through the gas supply pipe 210 connected to the down chamber 200 and supplying the gaseous reactant. The gas supply pipe 210 may include a plurality of spray nozzles 230 having a spray part 220 extending into the down chamber 200 and spraying a gaseous reactant to a lower side of the down chamber 200. The spray nozzle 230 may control a spray direction and a spray angle of the gaseous reactant to be sprayed, and as a means for applying a constant spray pressure, there is no particular limitation in the form and structure of the spray nozzle 230.

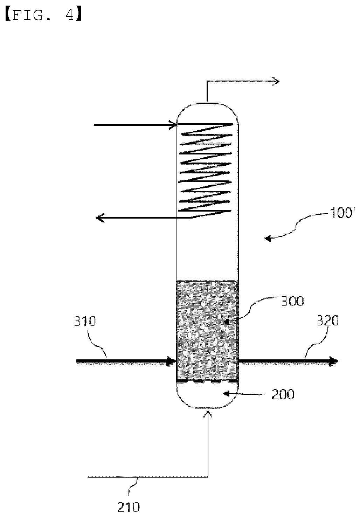

That is, in supplying a gaseous reactant such as gaseous ethylene to the down chamber 200, the gaseous reactant is sprayed and supplied downwardly toward a lower surface of the down chamber 200, so that the sprayed gaseous reactant flow collides with a sidewall or lower surface of the down chamber 200 to be changed and movement of the gaseous reactant toward the sidewall of the down chamber 200 may be induced. This may alleviate the central concentration of the gaseous reactant during an upward movement compared to the case of spraying the gaseous reactant upwardly toward the dispersion plate as in the related art shown in FIG. 4. That is, in the present invention, the gaseous reactant in a state of being uniformly mixed in the down chamber 200 may pass through the dispersion plate 350, thereby improving the mixing efficiency of the reaction zone 300 and, furthermore, inducing a uniform reaction over the entire reaction zone to improve a conversion rate.

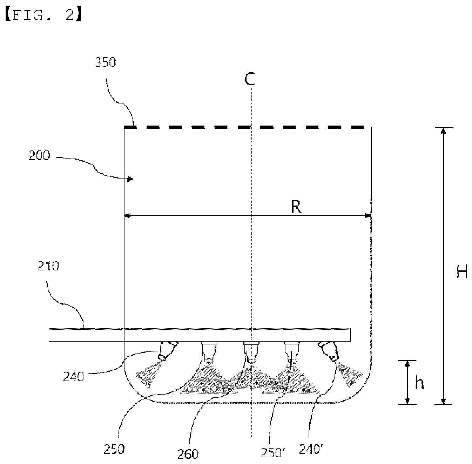

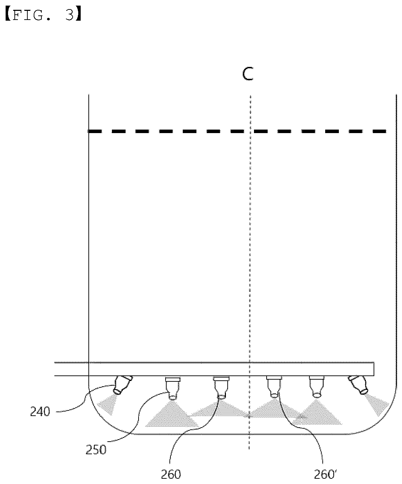

Referring to FIGS. 2 and 3, the gas supply pipe 210 and the spray part 220 may be configured as one integrated pipe. Meanwhile, the spray part 220 may extend from the gas supply pipe 210 into the interior of down chamber 200 in a horizontal direction of a horizontal cross-section (cross-section) of the down chamber 200. That is, the spray part 220 may horizontally extend through the center of the horizontal cross-section (cross-section) of the down chamber 200. In addition, the plurality of spray nozzles 230, 240, 250, 250', 260, and 260' provided in the spray part 220 may be arranged in a line in a diameter direction of the down chamber, that is, an extension direction of the spray part 220.

Meanwhile, the plurality of spray nozzles may have different spray directions and different spray angles according to positions.

That is, according to an embodiment of the present invention, the plurality of spray nozzles may include a first spray nozzle located in a central region of the down chamber 200, a second spray nozzle located in a region adjacent to the inner wall of the down chamber 200, and a third spray nozzle located in a region between the central region and a region adjacent to the inner wall of the down chamber 200.

Specifically, the first spray nozzle may be a spray nozzle closest to the center of the horizontal cross-section of the down chamber or to the center of the horizontal cross-section, and the second spray nozzle may be a spray nozzle closest to the inner wall of the down chamber. The third spray nozzle may be a spray nozzle located between the first and second spray nozzles.

More specifically, referring to FIG. 2, when the number of the plurality of spray nozzles provided in the spray part 220 is an odd number, the first spray nozzle 260 may be provided at a point at which a central axis C of the down chamber 200 intersects the spray part 220. Meanwhile, second spray nozzles 240 and 240' adjacent to the inner wall of the down chamber 200 may be provided, and third spray nozzles 250 and 250' may be provided between the first and second spray nozzles.

At this time, a spray direction of the first and third spray nozzles 250, 250', and 260 is a downward vertical direction, and an angle between the spray direction of the second spray nozzles 240 and 240' and the downward vertical direction may be 15° to 50°. Here, the spray direction refers to a direction of an outlet of the nozzle, in which a central axis of the nozzle is directed. That is, the spray directions of the first and third spray nozzles 250, 250' and 260 are directions toward a lower surface of the down chamber 200, and the second spray nozzles 240 and 240' have spray directions inclined toward the adjacent inner walls, respectively. This prevents stagnation of the gaseous reactant at the corresponding point through intensive spraying to the point at which the inner wall and the lower surface of the down chamber 200 meet, and induces a smoother flow of the gaseous reactant to maximize mixing efficiency.

According to an embodiment of the present invention, the plurality of spray nozzles may have different spray angles according to positions. Here, the spray angle refers to an angle formed by a region of the gaseous reactant discharged around the outlet of the spray nozzle. In this case, a spray angle of the first spray nozzle 260 may be 95° to 120°, a spray angle of the second spray nozzles 240 and 240' may be 15° to 45°, and a spray angle of the third spray nozzles 250 and 250' may be 50° to 90°. In spraying the gaseous reactant, the first spray nozzle 260 located in the central region of the down chamber 200 sprays with a relatively wide spray angle, and the second spray nozzles 240 and 240' adjacent to the sidewall of the down chamber 200 may spray with a narrow spray angle. Meanwhile, the third spray nozzles 250 and 250', which are between the first and second spray nozzles, may spray at a spray angle that is narrower than the spray angle of the first spray nozzle 260 and wider than the spray angle of the second spray nozzles 240 and 240'. When flow rates of the gaseous reactant discharged through the plurality of spray nozzles are the same or similar, the spray pressure of the second spray nozzles 240 and 240' having a narrow spray angle, compared to the first spray nozzle 260 having a wide spray angle, may be the largest.

Through this, by promoting mutual mixing by the flow of the gaseous reactant sprayed through the plurality of spray nozzles and inducing the formation of a vortex based on collision of the gaseous reactant with the lower and inner surfaces of the down chamber 200, mixing of the gaseous reactant may be further activated. Above all, by spraying the gaseous reactant at a narrow spray angle through the second spray nozzles 240 and 240' adjacent to the inner wall, central concentration of the gaseous reactant in the upward movement may be alleviated.

Meanwhile, referring to FIG. 3, when the number of the plurality of spray nozzles provided in the spray part 220 is an even number, the first spray nozzles 260 and 260' may be spaced apart from each other to correspond to each other based on the central axis C of the down chamber 200, as a center. Meanwhile, the second spray nozzle 240 adjacent to the inner wall of the down chamber 200 may be provided, and the third spray nozzle 250 may be provided between the first and second spray nozzles. Even when the number of the plurality of spray nozzles is an even number, the first to third spray nozzles may have the same spray directions and spray angles as those when the number of the plurality of spray nozzles is an odd number.

Referring to FIG. 2, in the bubble column reactor 100 according to an embodiment of the present invention, a height H of the down chamber 200 may be twice or more of a diameter R of the reactor, and specifically, the height H may be 2 to 3 times of the diameter R of the reactor. Here, the height H of the down chamber 200 is a length from the lower surface of the down chamber 200 to the dispersion plate 350. Compared to the bubble column reactor 100' of the related art shown in FIG. 4, the height H of the down chamber 200 is increased to be longer than twice of the diameter R of the reactor, thereby sufficiently securing a rising space of the gaseous reactant rising after being sprayed into the down chamber 200 and improving dispersion efficiency of the gaseous reactant. Meanwhile, by setting the height H of the down chamber 200 to be 3 times or less of the diameter R of the reactor, the size of the bubble tower reactor may be prevented from being excessively enlarged for a dispersion efficiency.

In addition, a ratio h/H of a height h of the spray part 220 to the height H of the down chamber 200 may be 0.1 to 0.5, specifically, 0.2 to 0.3. If the ratio of the height h/H is less than 0.1, the spray part 220 may be too close to the lower surface of the down chamber 200 to exert the effect of mixing efficiency properly by varying the spray directions and spray angles of the spray nozzles, and the flow of the sprayed gas may be negatively affected. Meanwhile, if the ratio h/H of the heights is greater than 0.5, the spray part 220 may be excessively spaced from the lower surface of the down chamber 200 and the sprayed gas may be switched to an upward flow, without contacting the lower surface of the down chamber 200, to result in a reduction in the mixing efficiency.

Meanwhile, as the gaseous reactant supplied to the reaction zone 300 of the bubble column reactor 100 passes through a liquid reaction medium in which a solvent and a catalyst are present, an oligomerization reaction proceeds through a catalytic reaction. In this case, the gaseous reactant and the reaction medium in the reaction zone 300 are mixed with each other to exist in two phases. The oligomer product generated through the oligomerization reaction of the reactant may be discharged through the product discharge line 320 as a liquid product effluent stream. A gaseous first effluent stream including unreacted monomers and solvents that have not undergone oligomerization in the reaction medium may be moved to a disengaging section 400 above the reaction zone 300.

Specifically, in the reactor 100, a polymer in a solid state is generated as a by-product in addition to the desired oligomer product due to the catalytic reaction of the monomers and floats in the liquid reaction medium. At this time, when a large amount of gaseous reactant including monomers is supplied to the reaction zone 300 through the dispersion plate 350, the solid polymer and the liquid solvent may be entrained together with the unreacted gaseous monomers and discharged as a first effluent stream.

As this first effluent stream passes through disengaging section 400, an upward movement rate may be reduced, and an entrained polymer and solvent may be readily removed within the first effluent stream.

Meanwhile, the first effluent stream passing through the disengaging section 400 may be introduced into a condensation zone 500 located above the disengaging section 400, and the solvent condensed in the condensation zone 500 may be refluxed to the reaction zone of the reactor through the disengaging section 400 to be reused in the oligomerization reaction. Meanwhile, a non-condensed gaseous component may be discharged to the outside of the bubble column reactor 100 through a gas discharge pipe 520.

In addition, the product effluent stream may include an oligomer product and a solvent produced through the oligomerization reaction, and the oligomer product and the solvent may be separated through an additional separation device. The separated solvent may be reused in the oligomer manufacturing process. Also, for example, when the oligomerization reaction is performed using an ethylene monomer as the monomer, the oligomer product may include 1-hexene and 1-octene.

Hereinabove, the bubble column reactor according to the present invention has been described and illustrated in the drawing. However, the description and the illustration of the drawing are for only essential components for understating the present invention, and processes and apparatuses not separately described and illustrated may be properly applicable and used for implementing the bubble column reactor according to the present invention, in addition to the processes and apparatuses described and illustrated in the description and drawing.

## Claims

1. A bubble column reactor comprising:
a down chamber formed at a lower portion;
a liquid reaction zone formed at an upper portion of the down chamber;
a dispersion plate formed between the down chamber and the reaction zone; and
a gas supply pipe connected to the down chamber to supply a gaseous reactant,
wherein the gas supply pipe includes a spray part extending into the down chamber,
wherein the spray part includes a plurality of spray nozzles spraying the gaseous reactant to a lower side of the down chamber.

2. The bubble column reactor of claim 1,
wherein the plurality of spray nozzles of the spray part are provided in a line in a diameter direction of the down chamber.

3. The bubble column reactor of claim 1,
wherein the spray part extends in a horizontal direction by way of a center of a horizontal cross-section of the down chamber.

4. The bubble column reactor of claim 1,
wherein a height of the down chamber is at least twice or more of a diameter of the reactor.

5. The bubble column reactor of claim 1,
wherein the plurality of spray nozzles includes:
a first spray nozzle located in a central region of the down chamber;
a second spray nozzle located in a region adjacent to an inner wall of the down chamber; and
a third spray nozzle located in a region between the central region and a region adjacent to the inner wall of the down chamber,
wherein spray directions of the first and third spray nozzles are a downward vertical direction, and
an angle between the spray direction of the second spray nozzle and the downward vertical direction is 15° to 50°.

6. The bubble column reactor of claim 5,
wherein the plurality of spray nozzles has different spray angles depending on locations.

7. The bubble column reactor of claim 6,
wherein a spray angle of the first spray nozzle is 95° to 120°,
a spray angle of the second spray nozzle is 15° to 45°, and
a spray angle of the third spray nozzle is 50° to 90°.

8. The bubble column reactor of claim 1,
Further comprising at least one or more reaction medium supply line connected to the reaction zone,
wherein the reaction medium includes a catalyst, a co-catalyst, and a solvent.

9. The bubble column reactor of claim 8,
further comprising a product discharge line connected to the reaction zone and provided on the other side of the reaction medium supply line.

10. The bubble column reactor of claim 1,
wherein the gaseous reactant includes ethylene.
